(19)

Europäisches
Patentamt
European
Patent Office
Office européen
des brevets

(11) **EP 2 454 595 B1**

(12) **EUROPEAN PATENT SPECIFICATION**

(45) Date of publication and mention
of the grant of the patent:
**02.01.2019 Bulletin 2019/01**

(21) Application number: **09847276.4**

(22) Date of filing: **11.09.2009**

(51) Int Cl.:
*G01N 33/573* (2006.01)    *A61B 5/01* (2006.01)
*A61M 25/06* (2006.01)

(86) International application number:
**PCT/IB2009/053988**

(87) International publication number:
**WO 2011/007205 (20.01.2011 Gazette 2011/03)**

(54) **TEMPERATURE-COMPENSATED IN-VIVO SENSOR**

TEMPERATURKOMPENSIERTER IN-VIVO-SENSOR

CAPTEUR IN VIVO COMPENSÉ EN TEMPÉRATURE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **15.07.2009 US 503376**

(43) Date of publication of application:
**23.05.2012 Bulletin 2012/21**

(73) Proprietor: **NOVA BIOMEDICAL CORPORATION
Waltham, MA 02454-9141 (US)**

(72) Inventors:
• **BICKOFF, Charles
Sharon
Massachusetts 02067 (US)**
• **PETERSON, Thomas H.
Wilmington
Massachusetts 01887 (US)**

• **JOBST, Gerhard
79108 Freiburg (DE)**

(74) Representative: **Katérle, Axel
Wuesthoff & Wuesthoff
Patentanwälte PartG mbB
Schweigerstraße 2
81541 München (DE)**

(56) References cited:
WO-A1-00/35340          WO-A2-2006/018425
US-A1- 2002 111 560     US-A1- 2005 183 954
US-A1- 2006 222 566     US-A1- 2006 293 576
US-A1- 2007 219 441     US-A1- 2008 086 041
US-A1- 2008 125 751     US-A1- 2008 125 751
US-A1- 2008 179 187     US-A1- 2009 143 658

**Description**

**BACKGROUND OF THE INVENTION**

1. Field of the Invention

The present invention relates generally to the field of medical devices. Particularly, the present invention relates to devices for placing a sensor at a selected site within the body of a patient. More particularly, the present invention relates to a temperature-compensated in-vivo sensor.

2. Description of the Prior Art

In the past, it was discovered that tight glycemic control in critically ill patients yielded statistically beneficial results in reducing mortality of patients treated in the intensive care unit for more than five days. A study done by Greet Van den Berghe and associates (New England Journal of Medicine, November 8, 2001) showed that using insulin to control blood glucose within the range of 80-110 mg/dL yielded statistically beneficial results in reducing mortality of patients treated in the intensive care unit for more than 5 days from 20.2 percent with conventional therapy to 10.6 percent with intensive insulin therapy. Additionally, intensive insulin control therapy reduced overall in-hospital mortality by 34 percent.

Attempts have been made in the past to monitor various blood analytes using sensors specific for the analytes being monitored. Most methods have involved reversing the direction of blood flow in an infusion line so that blood is pulled out of the patient's circulation at intervals, analyzed and then re-infused back into the patient by changing the direction of flow. A problem encountered in reversing an infusion line for sampling is determining how much blood should be withdrawn in order to be certain that pure, undiluted blood is in contact with the sensor.

U.S. Patent No. 5,165,406 (1992; Wong) discloses a sensor assembly for a combination infusion fluid delivery system and blood chemistry analysis system. The sensor assembly includes a sensor assembly with each of the assembly electrodes mounted in an electrode cavity in the assembly. The system includes provision for delivering the infusion fluid and measuring blood chemistry during reinfusion of the blood at approximately the same flow rates.

U.S. Patent No. 7,162,290 (2007; Levin) discloses a method and apparatus for periodically and automatically testing and monitoring a patient's blood glucose level. A disposable testing unit is carried by the patient's body and has a testing chamber in fluid communication with infusion lines and a catheter connected to a patient blood vessel. A reversible peristaltic pump pumps the infusion fluid forwardly into the patient blood vessel and reverses its direction to pump blood into the testing chamber to perform the glucose level test. The presence of blood in the testing chamber is sensed by a LED/photodetector pair or pairs. When the appropriate blood sample is present in the test chamber, a glucose oxidase electrode is energized to obtain the blood glucose level.

Although Levin discloses a method of halting the withdrawal of blood at the proper time so that a pure, undiluted sample is presented to the sensor, the method uses an expensive sensor and risks the possibility of contamination by the infusion process. Additionally, infusion of the flush solution has a diluting effect of the blood in the vicinity of the intravenous catheter and presents a time dependent function as to the frequency at which blood glucose can be measured.

It is also well-known that biosensors are typically calibrated to provide actual measurements at a specific temperature. Measurements obtained from a biosensor are dependent on the temperature of the surroundings. If the temperature of the surroundings changes, an error occurs in the measurement. An increase in temperature increases the slope of the curve of the biosensor and the computed analyte level is lower than the actual analyte level. On the other hand, a decrease in temperature decreases the slope of the curve, which causes the computed analyte level to be higher than the actual analyte level. Thus, a change in temperature of the surroundings causes an error in the computed analyte level.

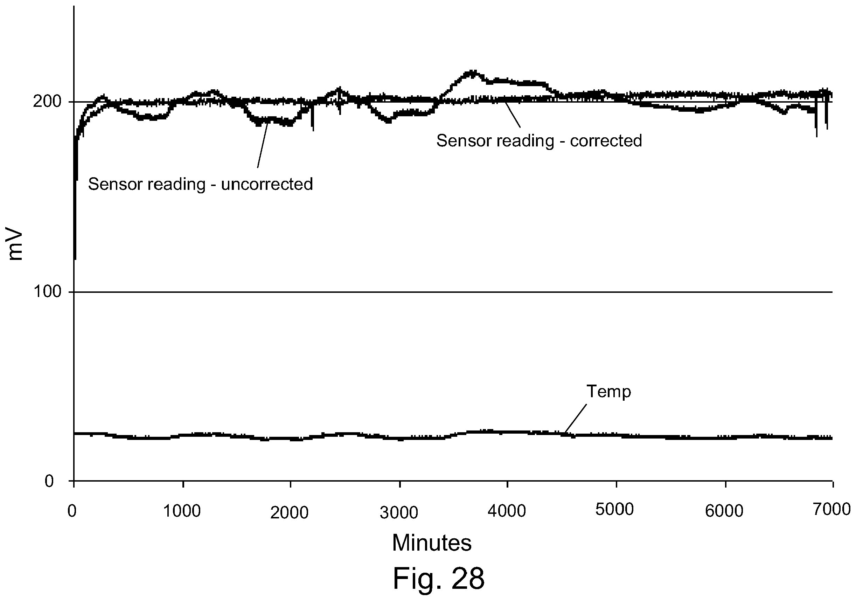

To compensate for temperature fluctuations, various statistical methods have been devised. Classical statistical methods are based on the sum of squared errors between the instrument and reference analyte measurements. Examples of these types of analyses are regression, analysis of variance and correlation. A disadvantage of these approaches is that they focus on the magnitude of measurement errors and do not distinguish those errors that would be clinically significant in the management of a disease such as diabetes. Error grid analysis was developed to classify measurement errors according to their perceived clinical significance. Figure 28 represents one such error grid analysis for glucose, which is called a Clark Error Grid. These errors are grouped into different levels or "zones" in order of assessed importance. Zone A represents clinically accurate measurements. Zone B represents measurements deviating from the reference glucose level by more than 20% but would lead to benign or no treatment. Zone C represents measurements deviating from the reference glucose level by more than 20% and would lead to unnecessary corrective treatment errors. Zone D represents measurements that are potentially dangerous by failing to detect and treat blood glucose levels outside of the desired target range. Zone E represents measurements resulting in erroneous treatment.

A modification to the error grid was later proposed by J. L. Parkes et al. ("A new consensus error grid to evaluate the clinical significance of inaccuracies in the measurement of blood glucose," Diabetes Care, 1997, 20:1034-6) to further discern the clinical relevance of glucose measurement errors. More recently, B. P. Kovatchev et al. ("Evaluating the accuracy of continuous glucose-monitoring sensors: continuous glucose-error grid analysis is illustrated by TheraSense Freestyle Navigator data," Diabetes Care, 2004, 27:1922-8), proposed an adaptation of error grid analysis for the evaluation of measurement error in the case of continuous glucose sensors.

Receiver operating characteristics (ROC) analysis has been used to assess the ability to detect hypoglycemia and hyperglycemia. In this approach, the sensitivity (percent of true events correctly classified) is compared to one minus the specificity (percent of non-events incorrectly classified). A commonly cited statistic from ROC analysis known as area under the curve (AUC) is commonly cited to describe how well a glucose meter or sensor detects values in the hypoglycemic and hyperglycemic range.

The accuracy of a glucose sensor is often summarized by reporting the percentage of values falling in zone A or B of an error grid, the correlation between sensor and reference glucose values and AUC values of hypoglycemia and hyperglycemia. However, these statistics do not adequately describe and may give inflated notions of the true accuracy of a glucose/analyte sensor. Currently analysis methods for accuracy of continuous glucose sensors focus on "point-by-point" assessments of accuracy and may miss important temporal aspects to the data. Even the proposed continuous error grid is a point-by-point assessment of pairs of consecutive glucose measurements.

US 2008/179187 discloses a sensing device provided on a substrate comprising a reference or counter electrode provided on a first side of the substrate, and a sensing electrode provided on a second side of the substrate. The working electrode may have membranes including heterocyclic nitrogen groups, and a fluid or gel sensing layer for facilitating sensing of the analyte including a catalyst. Alternatively, the sensing layer may be disposed in a polymeric or sol-gel matrix. The substrate may be flexible and may be a plastic or polymeric material. The reference electrode is an Ag/AgCl electrode. A temperature probe may be provided to the substrate. The sensors are formed by conductive traces having contact pads for connection to a control unit. A membrane encases the sensing device including the reference and the sensing electrodes.

US 2006/293576 discloses an analyte sensor comprising a conductive sensor provided on a substrate, which may be rigid or flexible. A working electrode, a counter electrode, and a reference electrode are provided to the sensor, which protrudes from an end of a delivery device. The sensor may be coated with a polymer, and may be further coated with a PHEMA polymer. The sensor may monitor a temperature for calibrating sensor values. Connection portions of the sensors may be connected to a monitor.

US 2008/086041 discloses an analyte monitor including a sensor having a substrate and a conductive trace. Contacts of the sensor connect to a monitor. A working electrode, a reference electrode, and a counter electrode are disposed on a surface of the substrate of the sensor. A sensing layer is disposed on the working electrode, and comprises a catalyst disposed within a polymeric or sol-gel matrix. A temperature probe may also be provided to the sensor on an opposite side of the sensor from the working electrode.

US 2002/111560 discloses an intravascular temperature sensor comprising a temperature sensor that is an RTD, a thermistor, a thermocouple, or a MEMS sensor. A catheter for delivering the sensor to an in-vivo location has a sensor disposed on an outer periphery of a distal end thereof.

US 2005/183954 discloses an implantable biosensor for measuring glucose. Electrical contacts of sensing elements contact a controller via a hub. A reference electrode is formed of cloridated silver. The sensors are formed as coils. Around the coils is disposed a first polysulfate layer, a second enzyme layer, and a third polymer membrane.

US 2007/219441 discloses an intravenous catheter including a biosensor having an active portion exposed through a sensing port formed in a distal portion of an outer wall of the catheter.

WO 2006/018425 A2 discloses a membrane system for a biosensor, the membrane comprising a continuous phase of one polymer and discrete domains of a second polymer with high oxygen permeability, where the polymers in each phase are immiscible.

Therefore, what is needed is a device that simplifies the measurement apparatus. What is also needed is a device that improves usability and limits the infusion fluid to the level required to clear the intravenous catheter site. What is further needed is a device that simplifies the procedures required of medical personnel to those closely related to existing accepted methods. What is still further needed is a device that accurately measures an analyte such as glucose when the sample temperature varies in real time during the measuring period.

## SUMMARY OF THE INVENTION

An in-vivo sensor assembly according to independent claim 1 is provided. It is an object of the present invention to provide a device that simplifies the components needed for the measurement apparatus. It is another object of the present invention to provide a device that improves usability and simplifies the procedures to those closely related to existing accepted method known to medical personnel. It is a further object of the present invention to provide a device that accurately measures an analyte in a sample fluid even when the sample fluid temperature varies in real-time during the measuring period.

The present disclosure achieves these and other objectives by providing a temperature-compensated, in-vivo biosensor. In one embodiment, the temperature-compensated, in-vivo sensor includes a sensor assembly having a sensor with a plurality of sensor elements at or near one end (i.e. the distal end), a sensor sheath containing the sensor and a hub connected to the other end of the sensor and/or sensor sheath (i.e. the proximal end). In another embodiment, the temperature-compensated, in-vivo sensor includes a sensor assembly and an insertion set. In still another embodiment, the temperature-compensated, in-vivo sensor includes a sensor assembly configured for use with commercially available catheter insertion devices. The sensor assembly includes a sensor sheath having a diameter substantially similar to a commercially available and conventional catheter insertion needle so that the sensor sheath sealingly engages the distal end of the catheter when the sensor assembly is inserted into the catheter after removal of the insertion needle.

In all embodiments of the present invention, the sensor sheath contains a sensor having a plurality of sensor elements disposed on a sensor shank adjacent a sensor distal end and electrical contacts at or adjacent a sensor proximal end. The plurality of sensor elements includes at least an analyte sensor element, a reference sensor element and a temperature sensor element. The temperature sensor element is a low resistive material such as a RTD sensor, a thermistor, a high resistive material such as amorphous germanium, or any device whose resistance changes with changing temperature. The sensor shank is sealingly embedded within the sensor sheath where the sensor elements are exposed at or adjacent the sensor distal end. The sensor may include one or more sensing elements on one side or on all sides of the sensor shank.

In some embodiments of the present invention, the sensor sheath includes a hub configured for mating with the luer fitting on a catheter. A secondary seal is made at the luer fitting.

The sensor signals are transmitted to a monitor by cabling or by radio waves. Optional signal conditioning electronics may be included to receive the sensor signals by way of electrical leads from the sensor. Either hard wiring or a radio link communicates the sensor signals to a monitor, which processes the sensor signals and displays temperature-compensated analytical values, trends and other patient related data for the measured analyte. A typical analyte is blood glucose. Blood glucose measurements are commonly used to determine insulin dosing in tight glycemic control protocols. Although blood glucose is an important blood component, other analytes are possible to measure within the constructs of the present invention.

According to the invention, there is disclosed an in-vivo sensor assembly for measuring an analyte in a fluid in a body. The sensor includes a sheath, a hub having a hub sheath portion and a hub cap connected to the hub sheath portion, and a sensor shank sealingly disposed within the sheath and having a shank distal end and a shank proximal end. The hub sheath portion is sealingly connected to a proximal end of the sheath and the hub cap has a connector receiver port.

The sensor shank includes a plurality of sensor elements at or adjacent the distal end of the in-vivo biosensor. The plurality of sensor elements includes at least an analyte sensor element for generating a signal in response to an analyte concentration in a body, a reference sensor element and a temperature sensor element for determining a temperature of an area adjacent to the analyte sensor element and for temperature compensating of an output of the analyte sensor element. The plurality of sensor elements are disposed adjacent the shank distal end and are exposed to the fluid of the body. The position of the temperature sensor relative to the analyte sensor element is critical for accurate analyte concentration measurements, as discussed later.

The sensor shank also includes a plurality of contact ears extending substantially parallel to the longitudinal axis of the sensor shank from the shank proximal end, the plurality of contact ears having electrical connector pads wherein the plurality of contact ears are offset from the sensor shank and from each other, the electrical connector pads being electrically coupled to the plurality of sensor elements; and an electrical connector having a shank connector board, the shank connector board being received and captured between the plurality of contact ears wherein the electrical connector and the shank proximal end are disposed within the hub cap. The offset spacing is configured so that the plurality of contact ears securely holds the connector board while insuring good electrical coupling between the electrical connector pads and the connector board.

The electrical connector pads are electrically coupled to the plurality of sensor elements. In another example according to the disclosure the sensor shank further includes an electrical connector having a shank connector board and an electrical connector receiver coupled to the shank connector board. The shank connector board is captured between the plurality of contact ears. When the shank connector board is captured by the contact ears, the connector pads of the plurality of contact ears are electrically coupled to the electrical connector receiver. The electrical connector and the shank proximal end are disposed within the hub cap such that the connector receiver is aligned with the connector receiver port in the hub cap.

In all embodiments of the present invention, the temperature sensor element is preferably a low-resistive material such as a RTD sensor element, a thermistor, a high-resistive material such as amorphous germanium and the like, or any device whose resistance changes with changing temperature. For a RTD sensor element, it is preferred to have a serially-connected, digitated array of a plurality of parallel and electrically conductive traces disposed on the sensor shank. The temperature sensor element is in thermal contact with the sensor elements and the fluid of the body.

One of the major advantages of the present invention particularly in embodiments configured for intravascular use is that the in-vivo sensor is structurally configured for use in combination with commercially-available IV catheters. This simplifies the procedure required of medical personnel since no additional special techniques are required for inserting the intravenous catheter. No highly specialized training is required since the procedures used by medical personnel to insert the intravascular or subcutaneous sensor are closely related to existing accepted methods. Upon removal of the insertion needle, the sensor assembly of the present invention is simply inserted and locked into place using the luer lock fitting. Because the present invention is configured for use with commercially-available IV catheters, no specially designed or customized insertion tools or devices are required to position the in-vivo sensor in the patient intravascularly. For subcutaneous applications, the use of a catheter is optional and the in-vivo sensor is not structurally restricted for use with and to fit within commercially-available catheters.

Another major advantage of the present invention is the inclusion of a temperature sensor for obtaining accurate analyte measurements. Biosensors are intrinsically sensitive to temperature. Relatively small changes in temperature can affect measurement results on the order of 3-4% per degree Celsius. Many clinical procedures benefit from tight glycemic control provided by an in-vivo continuous glucose monitoring (CGM) sensor. During these procedures, body temperature can fluctuate. In fact, many procedures involve dropping the core body temperature significantly. For example, it is customary during certain invasive thoracic procedures to "ice down" patients from 37° Celsius down to 25-30° Celsius. This induced hypothermia procedure intentionally slows certain autonomic responses. A sensor that is stable and calibrated at a body core temperature of 37° Celsius, is no longer calibrated nor accurate during such a procedure.

For CGM applications where the sensor is subcutaneously implanted approximately 5 to 8 millimeters into the abdomen (or other alternative locations), temperature changes can also have an adverse effect on system accuracy. Subcutaneous CGM patients are more likely healthy and highly mobile patients who may be moving in a changing variety of indoor and outdoor weather conditions. All of this may greatly affect the temperature at which the sensor is operating and, consequently, affecting the precision of the measurement readings that the sensor provides.

By placing a temperature sensing element in exact proximity to the biosensor in the blood flow for intravascular applications and in the tissue for subcutaneous applications, the temperature effect on the biosensor can be measured and the biosensor output can be properly compensated to reflect an accurate analyte concentration. An RTD sensor, preferably a platinum RTD, with a temperature accuracy of 0.1 °C is configured at the distal end of the sensor sheath. In fact, maintaining the temperature sensor within 0.25 mm of the analyte sensor greatly improves overall accuracy of the system.

In a further embodiment of the present invention, the analyte sensor element includes a analyte-selective reagent matrix having a plurality of layers where one of the plurality of layers contains an enzyme that is a substrate of the analyte to be measured and another layer disposed over the layer containing the enzyme that is a composite layer having a plurality of microspheres disposed in a hydrogel. The plurality of microspheres are made of a material having substantially little or no permeability to the analyte and substantially high permeability to oxygen while the hydrogel is made of a material that is permeable to the analyte. The material of the microspheres is preferably polydimethylsiloxane and the hydrogel is preferably one of polyurethane or poly-2-hydroxyethyl methacrylate (PHEMA). In another embodiment, the layer containing the enzyme is a PHEMA layer.

In still another embodiment of the present invention, the reagent matrix on the analyte sensor includes a hydrogel layer disposed on the composite layer. This hydrogel layer may optionally include a catalase. The hydrogel is preferably one of polyurethane or PHEMA.

In a further embodiment of the present invention, the reagent matrix on the analyte sensor includes a semi-permeable layer disposed between the composite layer and the electrically conductive electrode(s) of the analyte sensor.

In another example according to the disclosure there is disclosed a method of making an in-vivo analyte sensor having a base, a plurality of electrically conductive electrodes electrically coupled to a plurality of electrically conductive pathways, and an analyte-selective reagent matrix disposed on one of the plurality of electrically conductive electrodes. The reagent matrix is formed by disposing a plurality of layers on one of the electrically conductive electrodes where one layer is a composite layer formed by disposing a plurality of microspheres into a hydrogel and another layer containing an enzyme that is a substrate of the analyte to be measured is disposed between the electrically conductive electrode and the composite layer.

In another example according to the disclosure there is disclosed a method for temperature compensating an in-vivo analyte sensor measurement for an in-vivo sensor assembly having a plurality of sensor elements disposed at a distal end of a sensor sheath. The method includes measuring a current generated between an analyte sensor element and a reference sensor element, measuring an operating temperature using a temperature sensor element, determining an analyte concentration corresponding to the measured current, and adjusting the analyte concentration. The preferred algorithm for an in-vivo analyte sensor with an included temperature sensor element is analytically derived and empirically adjusted to provide very good correlation for all changes in analyte and temperature. One such algorithm is as follows:

$$C_{cor}r = E_{meas} \times R_{cal} \times (1-A(R_t) \times (1+B(E_{diff} \times R_{cal}))$$

where

$C_{corr}$ equals the temperature corrected analyte concentration;

$E_{meas}$ equals the measured potential (or current) of the analyte sensor;

$E_{diff}$ equals the difference between the measured potential of the analyte sensor and the calibrated potential of the analyte sensor;

$R_{cal}$ is a ratio of the calibrated analyte sensor concentration to the sensor potential;

$R_t$ is a ratio of the difference between the measured temperature and the temperature at calibration to the temperature at calibration;

A and B are constants

The term "$1-A(R_t)$" is a temperature correction component of the equation while the term "$1+B(E_{diff} \times R_{cal})$" is an analyte change component of the equation.

## BRIEF DESCRIPTION OF THE DRAWINGS

FIGURE 1 is a plan view showing the general installation of the intravenous catheter and sensor on a patient in a direct connection to the monitor.

FIGURE 2 is a plan view showing the general installation of the intravenous catheter and sensor on a patient in a radio communication connection to the monitor.

FIGURE 3 is a perspective view of one embodiment showing the intravascular sensor insertion set.

FIGURE 4 is an exploded view of the assembled sensor and cable shown in Fig. 3.

FIGURE 5 is an end view of the cable end of the hub showing the cross section of the sensor sheath.

FIGURE 6 is a perspective view of one embodiment of the sensor showing contact wings.

FIGURE 7 is an enlarged perspective view of the contact wings shown in Fig. 6.

FIGURE 8 is an enlarged perspective view showing the sensor element end in one embodiment of the sensor.

FIGURE 9 is an enlarged end view of the hub showing the connection between the cable and the connector end of the sensor.

FIGURE 10 is a perspective view of one embodiment showing the sensor assembly inserted into the intravenous catheter.

FIGURE 11 is a cross-sectional view of the sensor inserted into the intravenous catheter.

FIGURE 12 is an enlarged perspective view of one embodiment showing the sheath with a side opening/window exposing the sensor elements.

FIGURE 13 is an enlarged perspective view of another embodiment showing the sensor and sheath end with the intravenous catheter where all sensor elements are on one side.

FIGURE 14 is an enlarged cross-sectional view of the embodiment of the sensor assembly and intravenous catheter shown in Fig. 13.

FIGURE 15 is a perspective view of another embodiment showing the sensor assembly inserted into the intravenous catheter where the sensor elements extend beyond the end of the sensor sheath.

FIGURE 16 is an enlarged perspective view of the sensor elements shown in Fig. 15.

FIGURE 17 is a perspective view of an embodiment of the present invention showing an in-vivo sensor assembly.

FIGURE 18 is an exploded view of the sensor assembly shown in Fig. 17.

FIGURE 19 is an enlarged perspective view of the hub connector disposed between the contact wings of the sensor assembly shown in Fig. 18.

FIGURE 20 is an enlarged perspective view of the contact wings without the hub connector shown in Fig. 19.

FIGURE 21 is an enlarged perspective view of the sensor assembly showing the plurality of sensor elements and one embodiment of the temperature sensor element.

FIGURE 22 is an enlarged plan view of the temperature sensor element of the present invention showing the digitated array of one embodiment of the temperature sensor.

FIGURE 23 is an enlarged perspective view of the sensor assembly showing the plurality of sensor elements and another embodiment of the temperature sensor element.

FIGURE 24 is a perspective view of the sensor assembly showing temperature sensor element leads emerging from the proximal end of the sensor sheath.

FIGURE 25 is an enlarged, cross-sectional view of the sensor assembly shown in Fig. 24 with the sensor sheath.

FIGURE 26 is an illustration of one embodiment of the analyte sensor construction of the present invention.

FIGURE 27 is an illustration of a glucose sensor response showing the temperature, the uncorrected glucose response, the temperature-corrected glucose response and the glucose standard response for varying glucose concentrations and temperature.

FIGURE 28 is an illustration of a glucose sensor response to room temperature fluctuations over five days showing the temperature, the uncorrected glucose response, and the temperature-corrected glucose response.

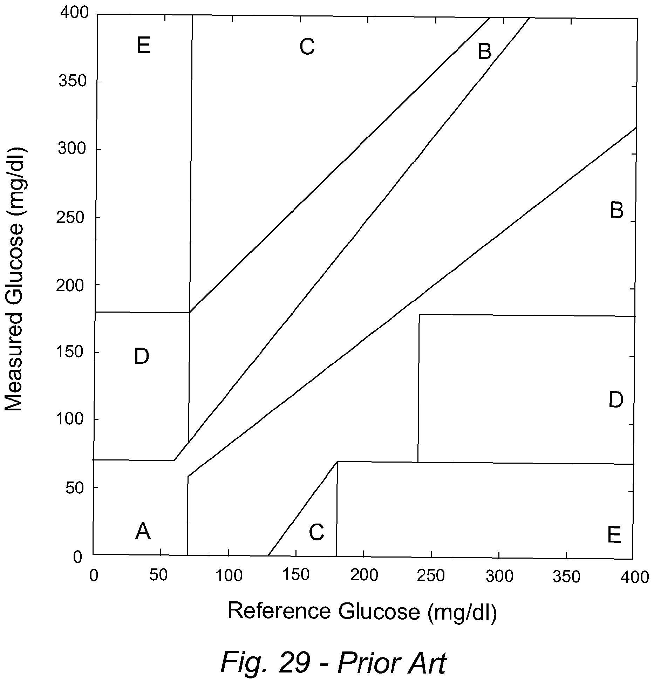

FIGURE 29 is a Clark Error Grid illustrating prior art glucose measurements without temperature compensation in relation to true glucose concentration values.

## DETAILED DESCRIPTION OF THE PREFERRED EMBODIMENT

Thermoregulation in humans is an important mechanism where the core temperature of the body can be regulated by adjustments in heat loss or heat retention mechanisms at the surface of the body. If the body core is too cold, and heat is to be retained, the body reacts by reducing vascular perfusion at the level of the skin (vasoconstriction) and increasing heat production through mechanisms such as shivering. If the body core is too warm, heat can be released by increasing the blood perfusion at the skin level (vasodilation) and through such mechanisms as sweating. These internal thermoregulation mechanisms are often initiated in combination with other active responses (e.g. adding clothing layers if cold, removing them if too warm) leading to complex, depth and time dependent, thermal gradients between surface and core temperatures that are not easily or accurately predicted by external or remote measurements. Because of thermoregulation caused by internal regulation, other active responses, or both, it is clear that significant thermal gradients exist between the skin, subcutaneous tissues, and body core temperatures. Therefore in the case of an analyte sensor whose performance is affected by temperature and where this performance can be corrected to improve measurement accuracy, the ability to measure temperature as close to the analyte sensing element as possible is of vital importance.

For in-vivo CGM, the measurement of fluctuating core body temperature is critical. As mentioned previously, commonly encountered factors such as surface heat loss, variable environmental conditions, base metabolic rate and daily cycles, medications, and other conditions (such as pregnancy) can increase the daily variability of core temperature significantly away from the stated normal of 98.6°F (37°C). In fact, standard normal daily temperature and individual variability in healthy persons can lead to core variations between 96°F and 100°F (36°F to 39°F). This variability can be increased further by medical conditions, intentional medical interventions, medications, fever, or severe environmental factors.

An individual's core temperature can be increased above normal in situations such as fever, disease, hyperthermia, etc., and can reach dangerous levels at 107°F (42°C). It is also not uncommon for patients suffering from hypothermia to have core temperatures in the 90°F (32°C) range. There are an increasing number of surgical procedures where the core temperature is intentionally lowered to improve surgical outcomes. These include the fields of neurology (e.g. for stroke recovery, aneurysm repair) and cardiovascular (e.g. bypass and other open heart surgical procedures). In these procedures, intra or extra vascular chillers can be used to reduce the core temperature to nearly 67°F (20°C).

For example, measuring glucose and maintaining tight glycemic control is essential to daily health and is especially critical in medical situations where an in-vivo (intravascular or subcutaneous) glucose sensor might be employed. An in-vivo glucose sensor will encounter a wide range of temperatures depending on the patient. For example, the temperature variation can be from 104°F (40 °C) and above for subjects in high fever to 67 °C (20 °C) for patients undergoing surgical procedures that require chilling. For precise temperature measurement and correction, the temperature must be measured as close to the glucose sensing element as possible.

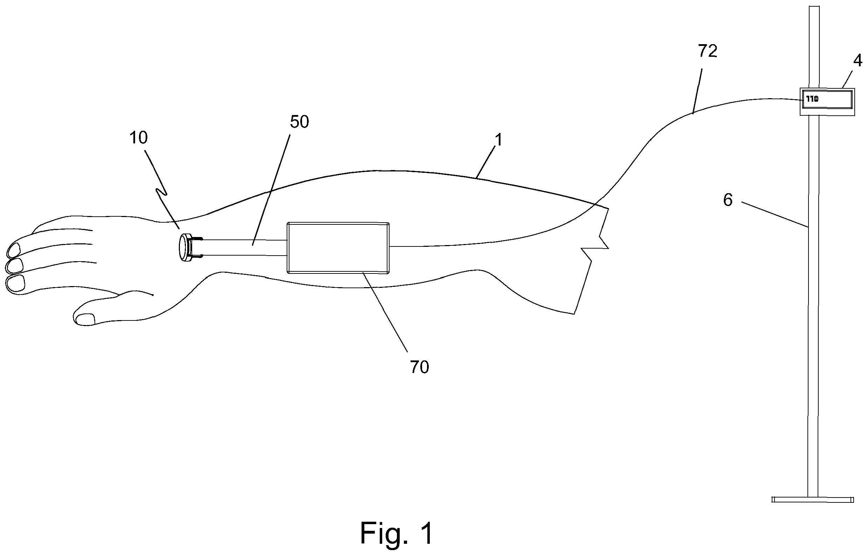

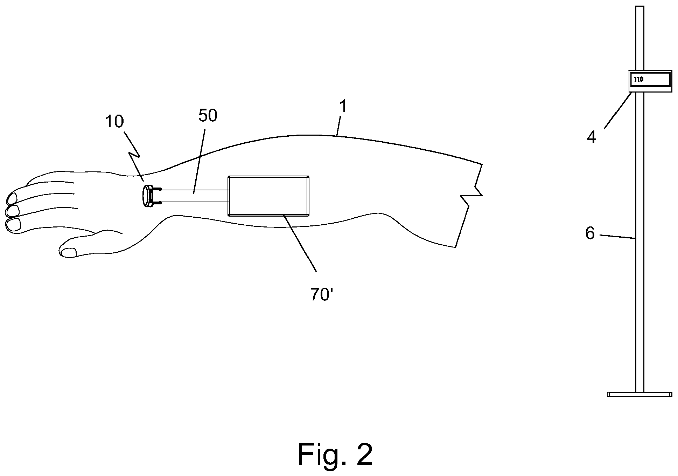

The embodiment(s) is illustrated in Figs. 1-28. Figures 1 and 2 illustrate the overall environment of the present invention connected to an arm 1 of a patient. Fig. 1 shows, by way of example, a disposable sensor assembly 30 of the present invention inserted into the intravascular system of the patient, which has been inserted into a vein on the back of arm 1 above the wrist. A conventional catheter assembly 20 (not shown) is preferably used with the present invention and together with the sensor assembly 30 make up one embodiment of the in-vivo sensor insertion set 10 of the present invention. Additionally, other locational installations on the patient are possible and often used.

As shown in Figure 1, a sensor cable 50 emanates from the sensor assembly 30 and is attached to a conditioning electronics and a cable junction unit 70. A monitor cable 72 electrically couples cable junction unit 70 to a monitor 4 mounted on a pole 6. Such poles as pole 6 are often used to mount electronic equipment as well as intravenous drips and the like. Another common location for the monitor 4 is the bed rail. Monitor cable 72 and sensor cable 50 transmit electrical signals generated by the sensor assembly 30 directly to monitor 4 where the signals are processed and displayed for access by medical personnel. Cable junction unit 70 is shown for convenience, as it is possible for monitor cable 72 and sensor cable 50 to be a single entity. It should be noted that other mounting configurations other than mounting monitor 4 to pole 6 is possible. For instance, it is possible to mount monitor 4 to a bed rail, cart mount, or other convenient location and often desirable.

Like the illustration in Fig. 1, Figure 2 shows a sensor cable 50 emanating from the sensor assembly 30 and attached to a conditioning electronics and radio unit 70'. The conditioning electronics and radio unit 70' transmits electrical signals generated by the sensor assembly 30 to the monitor 4 where the signals are processed and displayed for access by medical personnel.

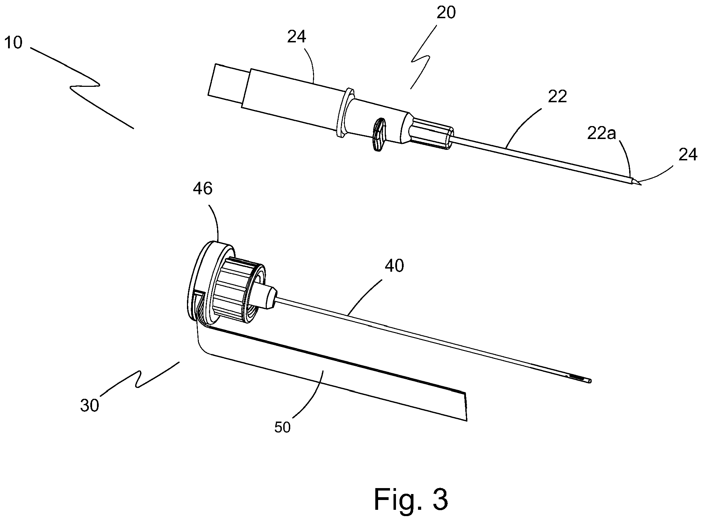

Turning now to Figure 3, there is illustrated one embodiment of the In-vivo sensor insertion set 10. Sensor insertion set 10 includes sensor assembly 30 and catheter assembly 20. Sensor assembly 30 includes a sensor sheath 40 sealingly connected to a sensor hub 46 from which sensor cable 50 extends. Catheter assembly 20 typically includes an insertion needle 24 disposed within a flexible catheter 22 and extends a predefined distance beyond a catheter distal end 22a. Sensor assembly 30 is preferably constructed to be insertable into a commercially available intravenous catheter assembly 20 that is typically available from a variety of medical suppliers. Some examples of these commercially available intravenous catheter assemblies include intravenous insertion catheters sold under the trademarks Introcan (manufactured by B. Braun) and Insyte Autoguard (manufactured by Becton Dickinson).

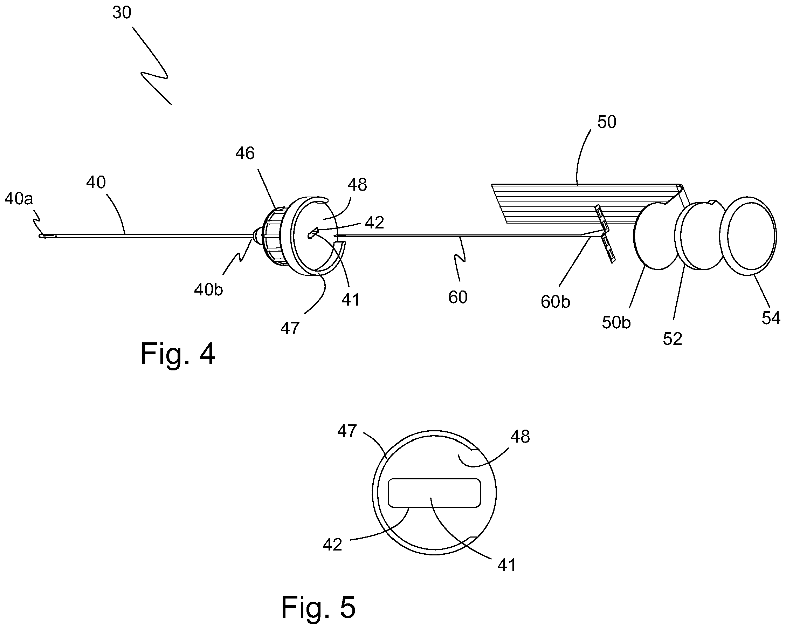

Figure 4 is an exploded view of sensor assembly 30 shown in Fig. 3. Sensor assembly 30 includes sensor sheath 40, sheath hub 46, a sensor 60, and sensor cable 50. Sensor sheath 40 includes a sheath distal end 40a and a sheath proximal end 40b. Sheath proximal end 40b is sealingly affixed to sheath hub 46. Sensor sheath 40 includes an internal channel 41 (not shown) that extends substantially the entire length of sheath 40 and receives sensor 60. Internal channel 41 of sheath 40 communicates with a hub port 42 in a hub surface 48. Sensor 60 has a shank proximal end 60b that is received within hub 46 against hub surface 48 along with a sensor cable proximal end 50b. Sensor 60 and cable proximal end 50b are fixedly retained within hub 46 by an electrical coupling means such as, for example, a pressure applying component 52 and a pressure cap 54. Pressure applying component 52 is optionally made from a resilient material such as a foam material that is placed over cable proximal end 50b to apply pressure between cable proximal end 50b and shank proximal end 60b. Pressure cap 54 provides the mechanism for maintaining the applied pressure and is preferably permanently affixed to hub 46.

Figure 5 is an enlarged plan view of hub surface 48. Internal channel 41 and hub port 42 have a cross-section that is suitable for receiving sensor 60 and can be any desired shape. Hub 46 optionally has a perimeter wall 47 around a major portion of the circumference of hub surface 48. Perimeter wall 47 facilitates attaching pressure cap 54 when capturing sensor 60, cable proximal end 50b and pressure applying component 52. Pressure cap 54 may be fixed to hub 46 by a snap fit, ultrasonic welding, chemical welding, and the like or by other means known to those of ordinary skill in the relevant art. Although cable 50 is shown as a flex circuit, it should be understood that other cable topologies are possible and usable.

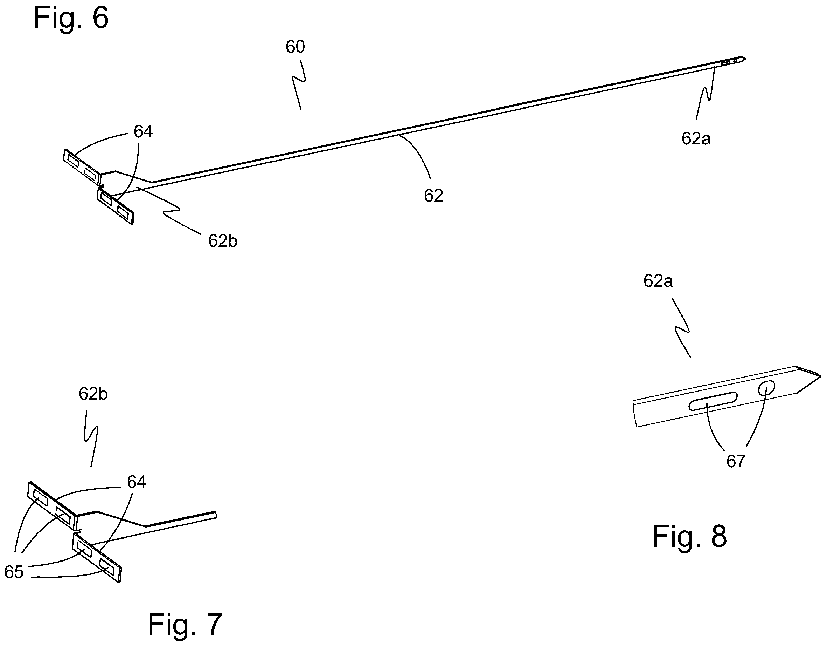

Figure 6 shows one example of sensor 60 according to the disclosure. Sensor 60 has a sensor shank 62 with a shank distal end 62a and shank proximal end 62b. Shank proximal end 62b has contact ears 64 that have been orthogonally folded outward from sensor shank 62. Contact ears 64 carry electrical contact pads thereon, which are more clearly illustrated in Figure 7. Turning now to Fig. 7 there is illustrated an enlarged view of shank proximal end 62b. Contact ears 64 have exposed thereon a plurality of electrical contact pads 65. By optionally configuring contact ears 64 as shown, electrical contact pads 65 are all facing in one direction facilitating connection to a single-sided sensor cable 50 such as a flex cable. Figure 8 is an enlarged view of shank distal end 62a. Shank distal end 62a has one or more sensor elements 67. Each of the one or more sensor elements 67 are electrically coupled to contact pads 65, typically by embedding one or more electrically conductive pathways (not shown) within sensor shank 62 where the electrically conductive pathways are electrically isolated from each other. In this particular embodiment, sensor elements 67 of sensor 60 are on both sides. Other quantities of electrical contacts and sensor elements are considered within the scope of the present disclosure.

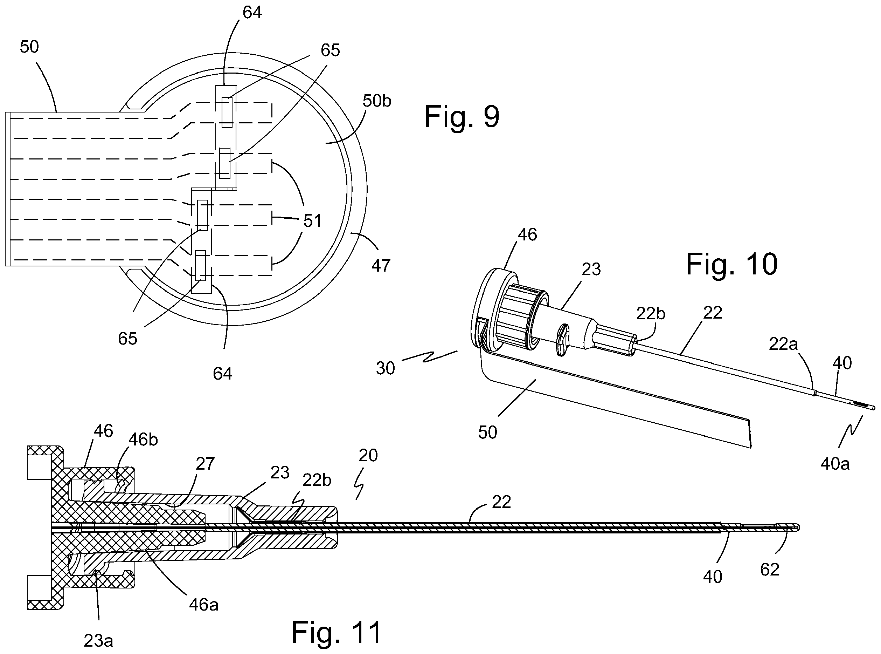

Turning now to Figure 9, there is illustrated an enlarged plan view of the electrical coupling assembly within hub 46. Cable 50 has a plurality of electrical conductors 51 that terminate at cable proximal end 50b. A portion of electrical conductors 51 are exposed and overlay against electrical contacts 65 of contact ears 64. As shown, cable proximal end 50b is preferably shaped to be captured within perimeter wall 47 of hub 46. As previously disclosed, pressure applying component 52 (not shown) is positioned on top of cable proximal end 50b. In this embodiment, pressure applying component 52 has a thickness greater than the height of perimeter wall 47 so that pressure cap 54, when installed, pushes pressure applying component 53 against cable proximal end 50b in order to maintain good electrical contact between electrical contacts 65 of contact ears 64 and the corresponding portions of exposed electrical conductors 51 at cable proximal end 50b.

Sensor assembly 30 positioned within catheter 22 is illustrated in Figure 10. Catheter 22 includes a luer fitting 23 attached permanently and hermetically to a catheter proximal end 22b to form a leak-proof entity. A catheter distal end 22a is tapered so that a liquid tight seal is formed between the inside diameter of catheter 22 and insertion needle 24 (not shown). The diameter of sensor sheath 40 is selected to be substantially the same as the diameter of insertion needle 24 so that, when sensor assembly 30 is inserted into catheter 22 after removal of insertion needle 24, a liquid tight seal is also formed at catheter distal end 22a between catheter distal end 22a and sensor sheath 40. As Fig. 10 illustrates, a sheath distal end 40a containing sensing elements 67 extends beyond catheter distal end 22a in order to expose sensing elements 67 to the sample fluid, i.e. the blood within the vein of the patient or in the subcutaneous fluid below the skin of the patient.

Luer fitting 23 (i.e. female luer fitting) removably connects to hub 46 of sensor assembly 30 in a similar fashion as standard luer-lock connections are used and known to those of ordinary skill in the art. Figure 11 is a cross sectional view which particularly shows the luer lock interface between the luer taper 46a of the sheath hub 46 (male luer fitting) and the luer taper 27 of the luer lock fitting 23 (female luer fitting) of the intravenous catheter assembly 20. The threads 23a of the luer lock fitting 23 of the intravenous catheter assembly 20 threadingly engages with the threads 46b of the sheath hub 46.

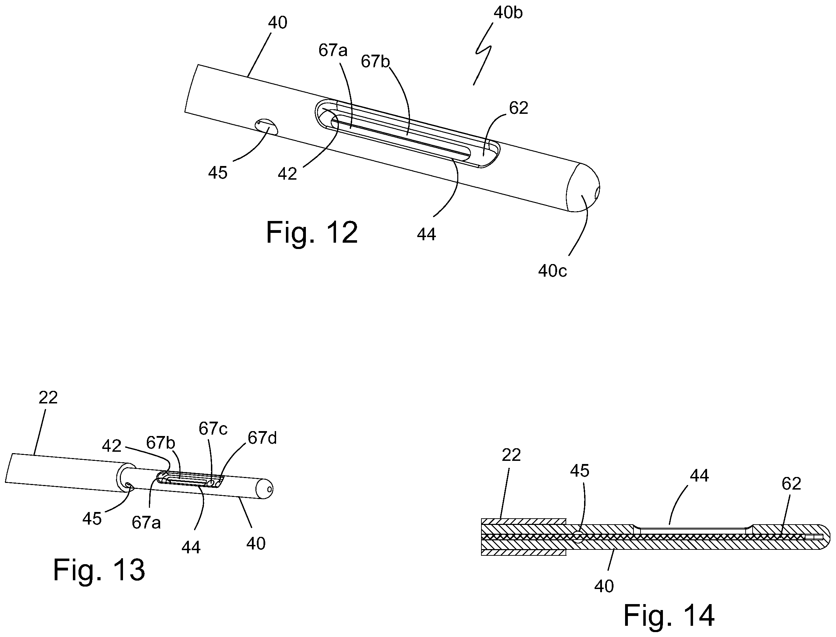

Turning now to Figure 12, there is illustrated an enlarged perspective view of one embodiment of the sensor elements 67 of sensor 60. Sensor sheath 40 has a side opening 44, i.e. a window, near sheath distal end 40b. Two sensor elements 67a, 67b on sensor shank 62 are disposed at side opening 44. In this embodiment, sheath distal end 40b has a sealed end 40c. Sensor sheath 40 also includes a cross-drilled opening 45 to provide access for disposing a sealant around sensor shank 62 and sheath channel 42 at sheath distal end 40b to form a liquid tight seal. It should be noted that sensor sheath 40 may optionally include additional side openings or windows to accommodate additional sensor elements to measure a plurality of blood analytes.

Figure 13 shows another embodiment of sensor assembly 30 where all sensor elements 67a, 67b, 67c, and 67d are on the same side of sensor shank 62. Sensor elements 67a, 67b, 67c, and 67d are positioned within sheath 40 to be located beneath sheath side opening 44. The size and/or shape of sensor elements 67a-d are illustrative only and may include more or less sensor elements in any shape configuration desired so long as the sensor elements are located at side opening 44. Sheath 40 also includes cross-drilled opening 45 for applying sealant around sensor shank 62 and sheath channel 42 to form a liquid tight seal. Figure 14 is a cross-section view of the embodiment in Fig. 13. Fig. 14 more clearly shows the relational detail of sensor shank 62, sheath side opening 44 and cross-drilled opening 45.

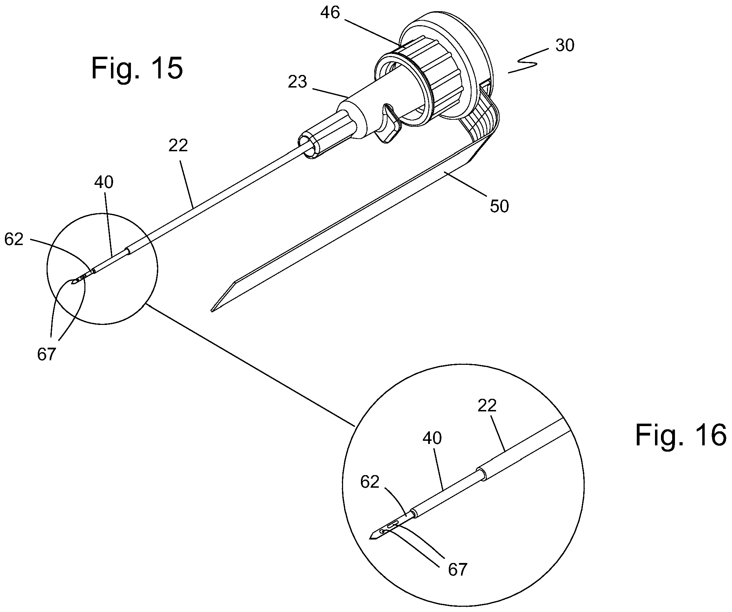

Figure 15 is a perspective view of another embodiment of the present invention. In this combination of sensor assembly 30 and catheter 40, sensor elements 67 are not protectively disposed beneath a window in sensor sheath 40 but positioned on a portion of sensor shank 62 that extends beyond sheath distal end 40b. Figure 16 is an enlarged detail view of the distal end of the embodiment in Fig. 15. Fig. 16 more clearly shows the relative relational detail between sensor elements 67, sensor shank 62, sensor sheath 40, and catheter 22.

Because sensor 60 is positioned within sensor sheath 40, sensor shank 62 may have a characteristic of being rigid or flexible or any degree of rigidity/flexibility. Preferably, sensor shank 62 is flexibly resilient to provide less susceptibility to damage during handling and use when configured for any embodiment of the present invention.

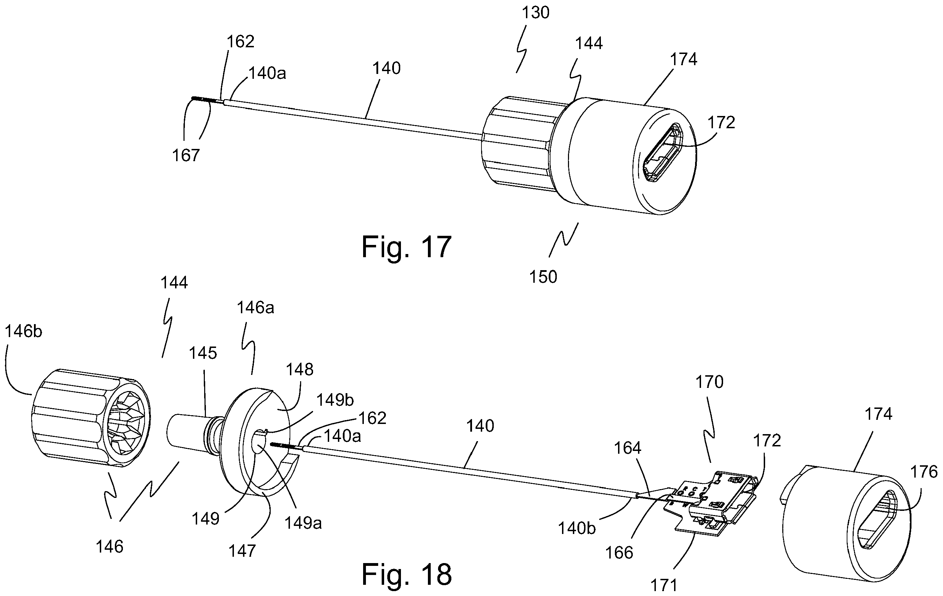

Turning now to Figure 17, there is illustrated an embodiment of the present invention. An in-vivo sensor assembly 130 is disclosed and includes a sheath 140, a sensor shank 160 (not shown) sealingly disposed within sheath 140 and a hub 150 sealingly coupled to sheath 140. In this embodiment, a shank distal end 162 extends beyond a sheath distal end 140a of sheath 140. It is noted, however, that in-vivo sensor assembly 130 may have other configurations as previously described. There is exposed a plurality of sensor elements 167 at shank distal end 162. Hub 150 includes a hub sheath portion 144 and a hub cap 174.

Figure 18 illustrates an exploded view of in-vivo sensor assembly 130. Sensor shank 160 is sealingly disposed within sheath 140 with distal end 162 extending from the sheath distal end 140a of sheath 140 and a proximal end 164 extending from a sheath proximal end 140b of sheath 140. Sheath 140 includes an internal channel 141 (not shown) that extends substantially the entire length of sheath 140 and receives sensor shank 160. Internal channel 141 of sheath 140 communicates with a hub port 149 in a hub surface 148. As in the previously disclosed embodiment, the plurality of sensor elements 167 includes at least an analyte sensor element for generating a signal in response to an analyte concentration in the fluid of the body, a reference sensor element and a temperature sensor element for determining the temperature of an area adjacent to the analyte sensor element, the area being the temperature of the analyte sensor and/or the temperature of the fluid of the body that is in contact with the analyte sensor element.

Proximal end 164 widens to form a plurality of contact ears 166. Connected to contact ears 166 is an electrical connector 170. Electrical connector 170 is received into and protected by hub cap 174. Electrical connector 170 includes a shank connector board 171 and an electrical connector receiver 172 that is physically and electrically coupled to shank connector board 171. Hub cap 174 includes a connector receiver port 176 that is positioned within the end of hub cap 174 to align with electrical connector receiver 172 when hub cap 174 is assembled to in-vivo sensor assembly 130. Hub sheath portion 144 includes a shank receiving enclosure 146a and a luer locking portion 146b. Shank receiving enclosure 146a includes a hub surface 148 with an optional perimeter wall 147 extending transversely around a major portion of hub surface 148. Extending away from and opposite hub surface 148 is a tubular portion 145. Tubular portion 145 has a central bore 149a for receiving sheath 140 and an optional notch 149b at hub surface 148 and extending laterally to central bore 149a for receiving part of widened portion 164 to prevent sensor shank 160 from rotating within central bore 149 during assembly. Luer lock portion (luer retention nut) 146b receives tubular portion 145 and is fixedly attached to tubular portion 145 forming luer lock portion 146. Luer lock portion 146 is a male luer fitting (hidden from view) that is structured to attach to a female luer fitting such as those commonly used on needles and catheters.

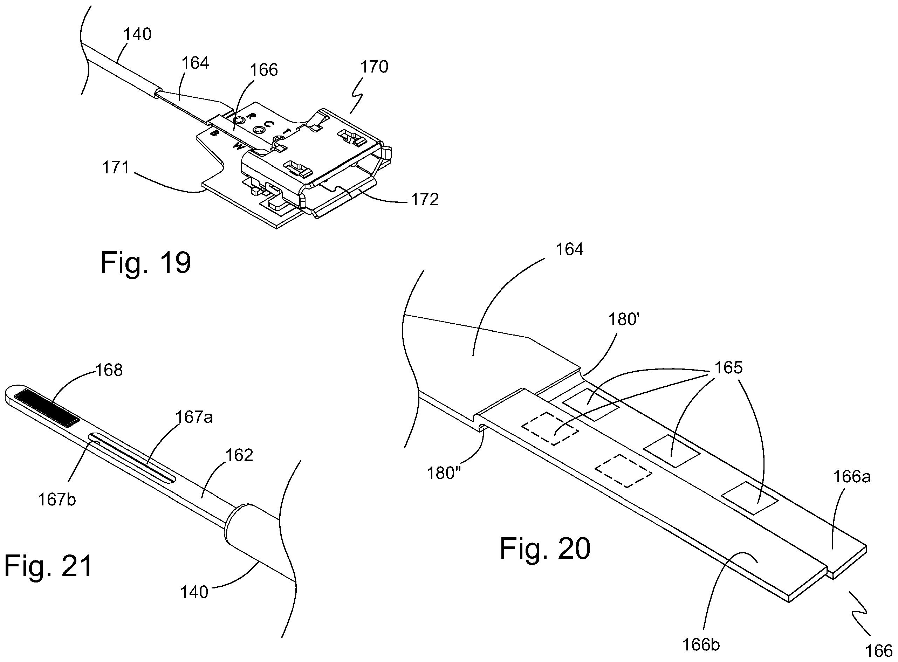

Figure 19 is an enlarged view of shank proximal end 164 extending from sheath 140. Fig. 19 more clearly shows the widened portion of shank proximal end 164 and the contact ears 166 that receive and capture shank connector board 171. In this embodiment as seen in Fig. 20, the plurality of contact ears 166 is offset from sensor shank 160. One of the contact ears indicated by reference number 166a is offset below the plane of shank proximal end 164 at 180'. The other of the illustrated contact ears indicated by reference number 166b is offset above the plane of shank proximal end 164 at 180". As shown, contact ears 166a and 166b have electrical connector pads 165. As can be seen, contact ears 166a and 166b are offset in such a way so that the connector pads 165 on each contact ear are spatially positioned to face towards the plane of sensor shank 160 and towards each other. The separation between contact ears 166a and 166b receives and captures shank connector board 171, which has corresponding electrical points of contact that coincide with connector pads 165 on contact ears 166a and 166b. Although only two contact ears 166a, 166b are shown, it is contemplated that additional contact ears may be included.

Figure 21 shows an enlarged view of one embodiment of sensor shank distal end 162. Sensor shank distal end 162 includes analyte sensor element 167a, reference sensor element 167b and temperature sensor element 168. It should be understood that temperature sensor element 168 may be located on either side of sensor shank 160, coaxially in front of or behind sensor elements 167, or on the side opposite sensor elements 167 so long as temperature sensor element 168 is in the proximate vicinity of sensor elements 167 in order to accurately record the temperature surrounding the sensor elements 167 and the fluid adjacent the sensor elements 167.

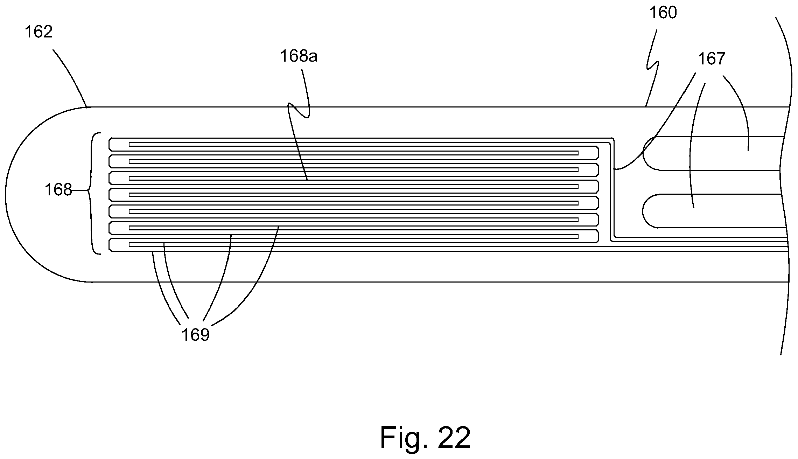

Turning now to Figure 22, there is illustrated one embodiment of a temperature sensor 168 for use in the present invention. Temperature sensor 168 may be one of the sensor elements 167a-d and connected to two of the electrical contacts 165 of contact ears 166. Alternatively, temperature sensor 168 may be attached to sensor sheath 140, located adjacent to sensor elements 167, co-located on the same plane as sensor elements 167, integrated into sensor elements 167, placed in the vicinity of sensor elements 167, placed at a location that is representative of the temperature around sensor elements 167, or placed in a location that tracks the temperature around sensor elements 167. Temperature sensor 168 measures the temperature at sensor elements 167 to compensate for any temperature fluctuation that would lead to inaccurate analyte readings. Temperature sensor 168 may be one of a thermistor, a resistance temperature detector (RTD), and the like. The temperature sensor illustrated in Fig. 22 is a RTD sensor. This type of temperature sensor exploits the predictable change in electrical resistance of some materials with changing temperature. Platinum is the preferred metal when making RTDs because of platinum's linear resistance-temperature relationship and its chemical inertness. In the preferred configuration of the RTD sensor, the RTD sensor has a digitated, serial array 168a made of a plurality of platinum arms or traces 169 disposed at distal end 162 of sensor shank 160 forming one of the sensor elements 167a-d. The size of temperature sensor 168 as illustrated is typically about 0.005 in. (0.127 mm) wide by about 0.010 in. (0.254 mm) long, but may be larger or smaller depending on the size of sensor shank 160 or on the capability of the measuring electronics to which in-vivo sensor assembly 130 is electrically coupled. A pair of electrical contacts 165 is electrically coupled to temperature sensor 168.

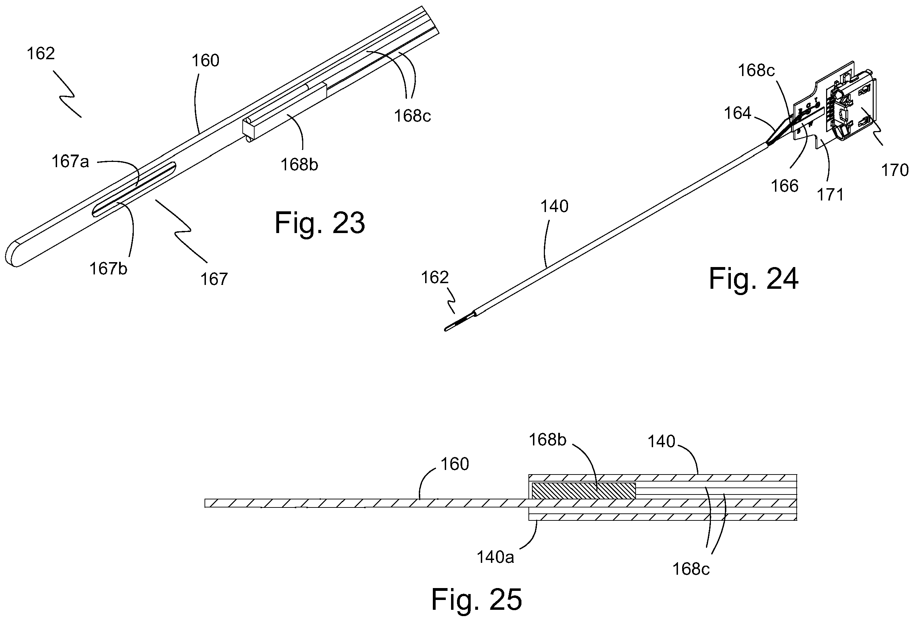

Turning now to Fig. 23, there is illustrated an alternative embodiment of the temperature sensor. Figure 23 shows an enlarged perspective view of one embodiment of sensor shank distal end 162. Sensor shank distal end 162 includes analyte sensor element 167a, a blank sensor element 167b and temperature sensor element 168. In this embodiment, a reference electrode and a counter electrode (not shown) are provided on the opposite side of sensor shank 160. It is contemplated that the sensor elements 167 may also all be configured on the same side of sensor shank 160, as previously disclosed. It is further contemplated that temperature sensor element 168 may be located on either side of sensor shank 160 so long as temperature sensor element 168 is in the proximate vicinity of sensor elements 167 in order to accurately record the temperature surrounding the sensor elements 167 and the fluid adjacent the sensor elements 167. To accurately record the temperature surrounding the sensor elements 167, temperature sensor element 68 must be no further than 0.25 mm from the working electrode containing the enzyme that is a substrate of the analyte intended to be measured. Although the accurate measurement of temperature at the sensor location is critical, this is extremely critical particularly in subcutaneous applications where the sensors are positioned approximately 5-8 mm below the skin and temperature fluctuation is more easily induced by room temperature. In this embodiment, the temperature sensor is a thermistor 168b. The preferred thermistor is a customized medical NTC thermistor manufactured by Adsem, Inc. of Palo Alto, CA. The thermistor preferably has 0.1 °C interchangeability but thermistors with 0.2 °C or 0.3 °C interchangeability may also be used.

Typically, thermistor 168b will have a pair of thermistor leads 168c with an insulating coating that is preferably about one micron thick. The insulating coating may also cover thermistor 168b. Alternatively, a separate sheath (not shown) may cover thermistor leads 168c or both thermistor 168b and thermistor leads 168c, which separate sheath may then be used to attach to sensor shank 160 and inserted within sensor sheath 140. Thermistor leads 168c may extend the length of sensor shank 160 and electrically couple to shank connector board 171 as is more clearly shown in Fig. 24. Fig. 24 shows thermistor leads 168c emerging from sensor sheath 140 at shank proximal end 164. Thermistor leads 168c may also be electrically coupled to a pair of electrical conductors 51 (not shown) of sensor shank 160, or the thermistor may be directly formed on and electrically coupled to the electrical conductors 51 embedded in the sensor shank 160, however, any change in resistance caused by the manufacturing/assembly method of the thermistor to the sensor shank 160 may require re-calibration of the thermistor. In an alternative embodiment, one of the temperature sensor leads shares the counter electrode sensor lead of sensor shank 160.

FIGURE 25 is an enlarged, cross-sectional view of thermistor 168b mounted on sensor shank 160. As illustrated, sensor sheath 140 covers and protects thermistor 168b. It should be understood, however, that thermistor 168b may be disposed on sensor shank 160 to extend beyond sheath distal end 140a.

Temperature compensation may be achieved by using a temperature compensation element that corrects for the error in the measurement recorded by the analyte sensor element due to a change in temperature. RTDs tend to have inconsistent interchangeability from one to another for purposes of measuring temperature and, thus, require either calibration of the RTD before use or an algorithm that compensates as best as possible for the interchangeability differences between RTD sensors. Thermistors, on the other hand, have very good interchangeability, are available with thermistor interchangeability of 0.1 °C, and can provide relatively accurate temperature measurement because of the interchangeability.

For sensor elements 167 made according to the embodiment of the present invention using an RTD sensor, temperature compensation may be expressed by the following algorithm without calibrating each RTD/sensor. The algorithm has been analytically derived and empirically adjusted to show excellent correction for all changes in analyte (and more particularly glucose) and temperature, given a starting calibration point referred to below as $R_{cal}$:

$$C_{cor}r = E_{meas} \times R_{cal} \times (1-A(R_t) \times (1+B(E_{diff} \times R_{cal}))$$

where,

$C_{corr}$ equals the temperature corrected analyte concentration;
$E_{meas}$ equals the measured potential (or current) of the analyte sensor;
$E_{diff}$ equals the difference between the measured potential of the analyte sensor and the calibrated potential of the analyte sensor;
$R_{cal}$ is a ratio of the calibrated analyte sensor concentration to the sensor potential;
$R_t$ is a ratio of the difference between the measured temperature and the temperature at calibration to the temperature at calibration;
A and B are constants

The term "$1-A(R_t)$" is a temperature correction component of the equation while the term "$1+B(E_{diff} \times R_{cal})$" is an analyte change component of the equation.

Constants A and B are analytically derived and empirically determined based on the configuration of the sensor elements 167. Thus, constants A and B may change as the structure and chemistry of sensor elements 167 changes.

It is contemplated that for use in measuring other analytes, the algorithm may be further analytically derived and empirically adjusted accordingly.

When using a thermistor, temperature compensation is more easily determined due to the interchangeability of the thermistors. A more simplified algorithm has been analytically derived and empirically adjusted to show excellent correction for all changes in analyte (and more particularly glucose) and temperature, given a starting calibration point referred to below as $R_{cal}$.

$$C_{corr} = E_{meas} \times R_{cal} \times ((1-C) \times T_{delta})$$

where

$C_{corr}$ equals the temperature corrected analyte concentration;
$E_{meas}$ equals the measured potential (or current) of the analyte sensor;
$R_{cal}$ is a ratio of the calibrated analyte sensor concentration to the sensor potential;
$T_{delta}$ equals the difference between the measured temperature and the temperature at calibration;
C is a constant.

The following is one example for fabricating a sensor 60 of the present invention and, more particularly, an analyte sensor.

*Sensor Fabrication*

Step 1. Obtain a sheet of polyimide film, preferably with a thickness of about 0.002 to 0.004 inches. One option to obtain such a polyimide film is to remove the copper layer from a sheet of polyimide flexible laminate available from E. I. du Pont de Nemours and Company, Cat. No. AP8525 under the trademark Pyralux®. Pyralux® AP double-sided, copper-clad laminate is an all-polyimide composite polyimide film bonded to copper foil. Chemical etching is the preferred method for removing the copper layer. The polyimide sheet will become the polyimide support substrate for the sensor elements 67 of the present invention.

Step 2. Apply liquid photoresist to both sides of the polyimide support substrate, expose the photoresist to UV light in a predefined pattern, and remove the unexposed areas to create a pattern for metal deposition. It should be understood that the preferred embodiment of the present invention has sensor elements 67 on both sides of the support substrate but that a single-sided sensor can also be made and is within the scope of the present invention. It is also understood that isolated electrically-conductive pathways are defined in the pattern between each sensor element 67 and a corresponding electrical contact 65. A single sheet of polyimide support substrate provides a plurality of sensors 60. Typically, one side contains the defined two electrodes per sensor (referred to as the top side) while the opposite side contains the reference and/or counter electrodes (referred to as the backside).

Step 3. Coat both sides with one or more layers of electrically conductive materials by vacuum deposition. Acceptable electrically conductive materials include platinum, gold, and the like. Preferably, platinum with a layer of titanium deposited thereon is used for the present invention. Platinum without the titanium layer is preferably used for forming the digitated, serial array 68a for temperature sensor 68.

Step 4. Remove the photoresist including the electrically conductive material on top of the photoresist surface leaving a pattern of electrically conductive material on the polyimide surfaces.

Step 5. Apply an insulation layer to both sides of the modified polyimide sheet preferably by lamination. The insulation layer is preferably a flexible photoimageable coverlay available from E. I. du Pont de Nemours and Company as Pyralux® PC. Pyralux® PC is a flexible, dry film solder mask used to encapsulate flexible printed circuitry. The dry film can be used as a solder mask by patterning openings using conventional printed circuit exposure and development processes. Unexposed areas can be developed off as explained in the technical information brochure provided by Dupont. For the present invention, Pyralux® PC 1015 was used. Expose the insulation layer to UV light and wash out the unexposed portions of the insulation layer. Thermally cure the remaining insulation layer/dry film. The cured remaining insulation layer serves as not only an insulation layer for the temperature sensor 68 and the electrically-conductive pathways between each sensor element 67 and a corresponding electrical contact 65 but also forms the wells to confine and contain the dispensed layers disclosed below for the analyte sensor(s).

Step 6. This and the remaining steps refer to the analyte sensor(s) only and not the temperature sensor 68. Remove the titanium in the areas exposed by the insulation layer using aqueous hydrofluoric acid, which also conveniently removes any surface contaminants from the previous process.

Step 7. Deposit silver onto the electrodes defined by the electrically conductive material pattern on the backside of the polyimide support substrate, and subsequently convert a portion to silver chloride to create a Ag/AgCl electrode, which will serve as counter and reference electrode.

Step 8. Deposit a semi-permeable membrane to the two electrodes per sensor defined on the top side (i.e. glucose electrode and blank electrode) by electropolymerization.

Step 9. Deposit a hydrogel membrane onto the Ag/AgCl counter and reference electrode on the backside of the sheet by dispensing a predefined amount of hydrogel membrane solution, followed by UV curing and washing.

Step 10. Deposit a poly-2-hydroxyethyl methacrylate (PHEMA) membrane precursor solution onto the two electrodes per sensor defined on the top side, UV cure, wash and dry. It should be understood by those skilled in the art that one of the two electrodes is a glucose electrode and, accordingly, the PHEMA membrane precursor solution for this electrode additionally contains a glucose enzyme, preferably glucose oxidase.

Step 11. Deposit a composite membrane precursor solution onto the glucose electrode and the blank electrode, UV cure and dry. The preparation of the composite membrane precursor solution will now be described. Microspheres are prepared from a material having substantially no or little permeability to glucose but a substantially high permeability to oxygen. The microspheres are preferably prepared from PDMS (polydimethylsiloxane). The microspheres are mixed with a hydrogel precursor that allows the passage of glucose. While polyurethane hydrogels work, a PHEMA precursor is preferred. The ratio of microspheres to hydrogel determines the ratio of the glucose to oxygen permeability. Thus, one of ordinary skill in the art can easily determine the ratio that enables the desired dynamic range of glucose measurement at the required low oxygen consumptions. It should be noted that if a polyurethane hydrogel is used, the membrane is cured by evaporating the solvent instead of using ultraviolet light.

Step 12. Optionally deposit additional PHEMA membrane precursor solution to the glucose and blank electrode, UV cure and dry. This optional step adds catalase that prevents release of hydrogen peroxide to the biological environment, reduces flow rate influence on sensor sensitivity and prevents direct contact of the microspheres surface to the biological environment.

Step 13. Cut the polyimide sheet into individual sensors 60.

The individual sensors 60 are then assembled into the sensor sheath 40 according to the preferred embodiments previously described.

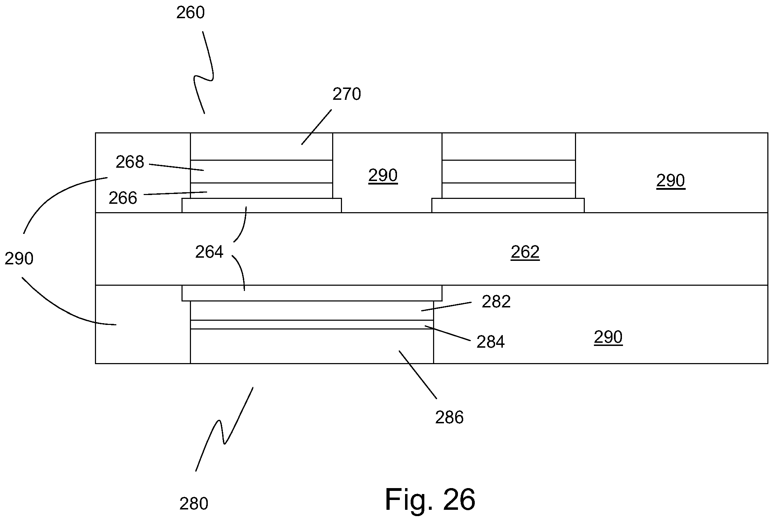

Figure 26 is an illustration showing an enlarged view of the sensor layers formed by the previously described procedure. As shown in Fig. 26, the sensor includes at least an analyte measuring electrode 260 and a reference electrode 280 formed on an insulating layer 290. The construction described above includes a base insulating layer 262 and an electrically conducting electrode 264 that are included in both analyte measuring electrode 260 and reference 280. Analyte measuring electrode 260 further includes a semi-permeable membrane or layer 266 over electrode 264. A hydrogel layer 268 containing an enzyme that is a substrate of the analyte to be measured is formed onto semi-permeable layer 266. Formed over the hydrogel layer 268 is composite layer 270. As described above, an optional hydrogel layer containing catalase (not shown) may be formed over composite layer 270.

Referecence electrode 280 includes a silver layer 282 formed over electrically conductive layer 264 and a silver-silver chloride layer 284 formed over silver layer 282. Formed over silver-silver chloride layer 284 is a PHEMA or urethane layer 286.

## Example 1

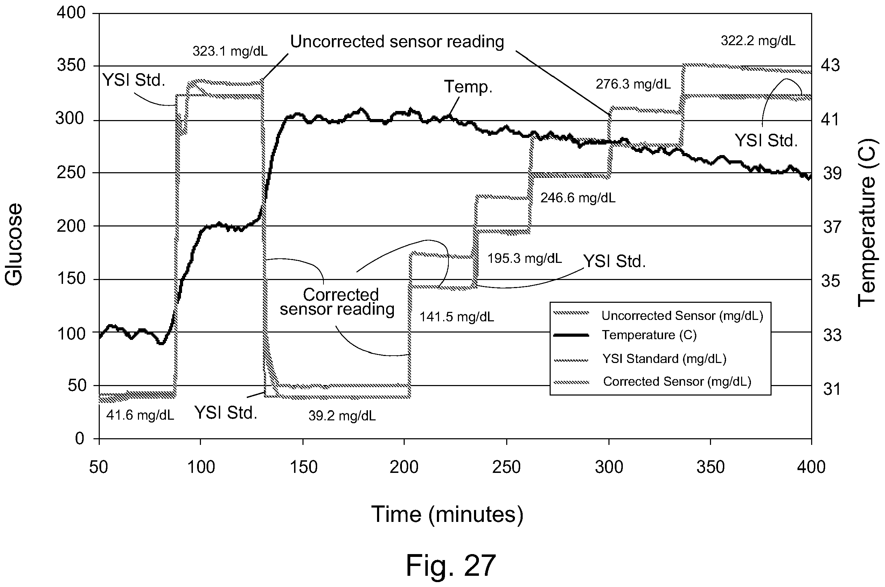

An example of experimental data with and without temperature correction using one embodiment of the present invention is illustrated in Figure 27. In this in-vitro example, a glucose sensor is exposed to a variety of glucose concentrations while at the same time the temperature of the environment is altered. The glucose concentrations are depicted in Fig. 27 adjacent the measurement traces.

Temperature is depicted on the right axis and shows an initial temperature of approximately 33 °C until approximately 80 minutes into the test. Thereafter, the temperature is gradually raised to 37 °C. After equilibrating at this new temperature point, the temperature is raised to 41 °C where it remains for approximately 60 minutes and then allowed to cool gradually. At the same time the temperature is altered, the sensor is exposed to several glucose concentrations (ranging from 39.2 mg/dl to 323.1 mg/dl), and the response of the glucose sensor is recorded. Glucose concentration is presented on the left axis. In an ideal sensor, the output of the sensor would precisely correlate with the concentration of the glucose (as confirmed by the YSI standard). The YSI standard is the glucose concentration of the same sample as measured with a YSI glucose analyzer (Model 2300 Stat Plus, YSI Inc., Yellow Spring, OH). However, temperature is known to affect sensor performance. Figure 27 displays the precise glucose concentrations (YSI Standard), the thermally uncorrected data (Uncorrected Sensor), and the sensor data corrected with the algorithm listed above. It is clear from the data, correction of temperature variability improves the accuracy of the glucose measurement. In fact, the data indicates the near-perfect compensation of the glucose measurement with that of the YSI standard when the uncorrected data is corrected using real-time temperature measurement with an RTD sensor element and the above-listed algorithm. As can be seen in Fig. 27, the corrected sensor reading tracing is nearly superimposed on the YSI standard tracing.

## Example 2

Even small fluctuations in temperature can result in glucose measurement variability and should be corrected if one is to present accurate glucose data to the user. In Figure 28, there is illustrated data obtained from an in-vitro test when a glucose sensor of the present invention having an integrated temperature sensor is placed in a vial of known glucose concentration and monitored for 5 days. The vial contained an aqueous standard solution having a glucose concentration of 280 mg/dl. Small fluctuations in room temperature are recorded by the temperature sensor and are also reflected in the performance of the glucose sensor. As shown by the data, small temperature fluctuations cause relatively large sensor reading fluctuations, which provide inaccurate concentration readings. By using temperature correction algorithms along with placement of the temperature sensor within 0.25 mm or closer to the enzyme measuring electrode, the temperature sensor data can be used to correct the glucose sensor performance for thermally induced fluctuations and provide an accurate reading to the user. This is clearly illustrated in Fig. 28.

Although the preferred embodiments of the present invention have been described herein, the above description is merely illustrative. Further modification of the invention herein disclosed will occur to those skilled in the respective arts and all such modifications are deemed to be within the scope of the invention as defined by the appended claims.

## Claims

**1.** An in-vivo sensor assembly for measuring an analyte in a fluid in a body, the sensor assembly comprising:

a sheath (140);
a hub (150) having a hub sheath portion (144) and a hub cap (174) connected to the hub sheath portion, the hub sheath portion (144) sealingly connected to a proximal end of the sheath, the hub cap (174) having a connector receiver port (172); and
a sensor shank (160) sealingly disposed within the sheath (140) and having a shank distal end (162) and a shank proximal end (164), the sensor shank (160) comprising:

a plurality of sensor elements (167) including at least an analyte sensor element (167a) for generating a signal in response to an analyte concentration in a body, a reference sensor element (167b) and a temperature sensor element (168) for determining a temperature of an area adjacent to the analyte sensor element (167a) and for compensating for an output of the analyte sensor element (167a), the plurality of sensor elements disposed adjacent the shank distal end (162) and exposed to the fluid of the body;
a plurality of contact ears (166) extending substantially parallel to the longitudinal axis of the sensor shank (160) from the shank proximal end (164), the plurality of contact ears (166) having electrical connector pads (165) wherein the plurality of contact ears (166) are offset from the sensor shank (160) and from each other, the electrical connector pads (165) being electrically coupled to the plurality of sensor elements; and
an electrical connector (170) having a shank connector board (171), the shank connector board (171) being received and captured between the plurality of contact ears (166) wherein the electrical connector (170) and the shank proximal end (164) are disposed within the hub cap (174).

**2.** The in-vivo sensor assembly of Claim 1 wherein the plurality of sensor elements and the shank distal end (162) are exposed to the fluid of the body at a location selected from the group consisting of beyond the sheath distal end (140a), at an opening in the sheath adjacent the sheath distal end (140a), and at orthogonal openings on opposite sides of the sheath adjacent the sheath distal end.

**3.** The in-vivo sensor assembly of Claims 1 wherein the temperature sensor element (168) is one of a resistance temperature detector or a thermistor.

**4.** The in-vivo sensor of Claim 3 wherein the resistance temperature detector is a serially-connected digitated array of a plurality of parallel and electrically conductive traces.

## Patentansprüche

**1.** In-vivo-Sensoranordnung zum Messen eines Analyten in einem Fluid in einem Körper, wobei die Sensoranordnung umfasst:

eine Hülse (140);
eine Nabe (150) mit einem Nabenhülsenabschnitt (144) und einer mit dem Nabenhülsenabschnitt verbundenen Nabenkappe (174), wobei der Nabenhülsenabschnitt (144) abdichtend mit einem proximalen Ende der Hülse verbunden ist und wobei die Nabenkappe (174) einen Verbinderempfängeranschluss (172) aufweist; und
einen Sensorschaft (160), der abdichtend innerhalb der Hülse (140) angeordnet ist und ein distales Schaftende (162) und ein proximales Schaftende (164) aufweist, wobei der Sensorschaft (160) umfasst:

eine Mehrzahl von Sensorelementen (167), die zumindest ein Analytsensorelement (167a) zum Erzeugen eines Signals in Abhängigkeit von einer Analytkonzentration in einem Körper, ein Referenzsensorelement (167b) und ein Temperatursensorelement (168) zum Ermitteln einer Temperatur eines Bereichs neben dem Analytsensorelement (167a) und zum Kompensieren eines Ausgangs des Analytsensorelements (167a) umfasst, wobei die Mehrzahl von Sensorelementen neben dem distalen Schaftende (162) angeordnet ist und dem Fluid des Körpers ausgesetzt ist;
eine Mehrzahl von Kontaktohren (166), die sich im Wesentlichen parallel zu der Längsachse des Sensorschafts (160) von dem proximalen Schaftende (164) erstrecken, wobei die Mehrzahl von Kontaktohren (166) elektrische Anschlussflächen (165) aufweist, die Mehrzahl von Kontaktohren (166) gegenüber dem Sensorschaft (160) und gegeneinander versetzt ist und die elektrischen Anschlussflächen (165) elektrisch mit der Mehrzahl von Sensorelementen verbunden sind; und

einen elektrischen Verbinder (170) mit einer Schaftverbindungsplatte (171), wobei die Schaftverbindungsplatte (171) zwischen der Mehrzahl von Kontaktohren (166) aufgenommen und festgehalten ist, wobei der elektrische Verbinder (170) und das proximale Schaftende (164) innerhalb der Nabenkappe (174) angeordnet sind.

**2.** In-vivo-Sensoranordnung nach Anspruch 1, wobei die Mehrzahl von Sensorelementen und das distale Schaftende (162) dem Fluid des Körpers an einer Stelle ausgesetzt sind, die aus der Gruppe ausgewählt ist, die aus jenseits des distalen Hülsenendes (140a), an einer Öffnung in der Hülse neben dem distalen Hülsenende (140a) und an orthogonalen Öffnungen auf gegenüberliegenden Seiten der Hülse neben dem distalen Hülsenende besteht.

**3.** In-vivo-Sensoranordnung nach Anspruch 1, wobei das Temperatursensorelement (168) ein Widerstandstemperaturfühler oder ein Thermistor ist.

**4.** In-vivo-Sensor nach Anspruch 3, wobei der Widerstandstemperaturfühler eine in Reihe geschaltete fingerartige Anordnung einer Mehrzahl paralleler und elektrisch leitfähiger Leiterbahnen ist.

**Revendications**

**1.** Ensemble de capteur in vivo pour mesurer un analyte dans un fluide dans un corps, l'ensemble de capteur comprenant :

une gaine (140) ;

un raccord (150) ayant une partie (144) de gaine de raccord et un bouchon (174) de raccord connecté à la partie de gaine de raccord, la partie (144) de gaine de raccord connectée de manière étanche à une extrémité proximale de la gaine, le bouchon (174) de raccord ayant un orifice (172) de récepteur de connecteur ; et

une tige (160) de capteur disposée de manière étanche à l'intérieur de la gaine (140) et ayant une extrémité distale (162) de tige et une extrémité proximale (164) de tige, la tige (160) de capteur comprenant :

une pluralité d'éléments (167) de capteur incluant au moins un élément (167a) de capteur d'analyte pour générer un signal en réponse à une concentration d'analyte dans un corps, un élément (167b) de capteur de référence et un élément (168) de capteur de température pour déterminer une température d'une zone adjacente à l'élément (167a) de capteur d'analyte et pour compenser une sortie de l'élément (167a) de capteur d'analyte, la pluralité d'éléments de capteur disposés adjacents à l'extrémité distale (162) de tige et exposés au fluide du corps ;

une pluralité de pattes de contact (166) s'étendant sensiblement parallèles à l'axe longitudinal de la tige (160) de capteur depuis l'extrémité proximale (164) de tige, la pluralité de pattes de contact (166) ayant des plots (165) de connecteur électrique, dans lequel la pluralité de pattes de contact (166) sont décalées par rapport à la tige (160) de capteur et les unes par rapport aux autres, les plots (165) de connecteur électrique étant électriquement couplés à la pluralité d'éléments de capteur ; et

un connecteur électrique (170) ayant une carte (171) de connecteur de tige, la carte (171) de connecteur de tige étant reçue et capturée entre la pluralité de pattes de contact (166), dans lequel le connecteur électrique (170) et l'extrémité proximale (164) de tige sont disposés à l'intérieur du bouchon (174) de raccord.

**2.** Ensemble de capteur in vivo selon la revendication 1, dans lequel la pluralité d'éléments de capteur et l'extrémité distale (162) de tige sont exposés au fluide du corps en un emplacement choisi parmi le groupe consistant en au-delà de l'extrémité distale (140a) de gaine, au niveau d'une ouverture dans la gaine adjacente à l'extrémité distale (140a) de gaine, et au niveau d'ouvertures orthogonales sur des côtés opposés de la gaine adjacentes à l'extrémité distale de gaine.

**3.** Ensemble de capteur in vivo selon la revendication 1, dans lequel l'élément (168) de capteur de température est un parmi un détecteur de température à résistance ou un thermistor.

**4.** Ensemble de capteur in vivo selon la revendication 3, dans lequel le détecteur de température à résistance est une matrice digitée connectée en série d'une pluralité de tracés parallèles et électro-conducteurs.

Fig. 1

Fig. 2

Fig. 3

Fig. 4

Fig. 5

EP 2 454 595 B1

Fig. 6

60

64

62a

62

62b

Fig. 7

62b

64

65

62a

67

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 21

Fig. 20

EP 2 454 595 B1

160

167

168a

162

168

169

Fig. 22

Fig. 23

Fig. 24

Fig. 25

EP 2 454 595 B1

Fig. 26

Fig. 27

EP 2 454 595 B1

Fig. 28

*Fig. 29 - Prior Art*

EP 2 454 595 B1

## REFERENCES CITED IN THE DESCRIPTION

**Patent documents cited in the description**

- US 5165406 A, Wong
- US 7162290 B, Levin
- US 2008179187 A
- US 2006293576 A
- US 2008086041 A
- US 2002111560 A
- US 2005183954 A
- US 2007219441 A
- WO 2006018425 A2

**Non-patent literature cited in the description**

- **GREET VAN DEN BERGHE.** *New England Journal of Medicine,* 08 November 2001
- **J. L. PARKES et al.** A new consensus error grid to evaluate the clinical significance of inaccuracies in the measurement of blood glucose. *Diabetes Care,* 1997, vol. 20, 1034-6
- **B. P. KOVATCHEV et al.** Evaluating the accuracy of continuous glucose-monitoring sensors: continuous glucose-error grid analysis is illustrated by Thera-Sense Freestyle Navigator data. *Diabetes Care,* 2004, vol. 27, 1922-8